(19) Europäisches Patentamt
European Patent Office
Office européen des brevets

(11) Publication number: **0 536 402 A1**

(12) # EUROPEAN PATENT APPLICATION published in accordance with Art. 158(3) EPC

(21) Application number: **91911965.1**

(51) Int. Cl.[5]: **C07D 211/46**, A61K 31/445

(22) Date of filing: **27.06.91**

(86) International application number: **PCT/JP91/00866**

(87) International publication number: **WO 92/00277 (09.01.92 92/02)**

(30) Priority: **29.06.90 JP 173629/90**
**04.02.91 JP 35546/91**

(43) Date of publication of application: **14.04.93 Bulletin 93/15**

(84) Designated Contracting States: **AT BE CH DE DK ES FR GB GR IT LI LU NL SE**

(71) Applicant: **NIPPON SHINYAKU COMPANY, LIMITED**
**14, Kisshoin Nishinosho Monguchicho**
**Minami-ku Kyoto-shi Kyoto 601(JP)**

(72) Inventor: **EZURE, Yohji**
**2-21-22, Nogohara**
**Otsu-shi, Shiga 520-21(JP)**
Inventor: **MARUO, Shigeaki**
**2-2 13-104, Minamiai**
**Ibaraki-shi, Osaka 567(JP)**
Inventor: **MIYAZAKI, Katsunori**
**32-35, Tsukigaoka 3-chome**
**Morioka-shi, Iwate 020-01(JP)**
Inventor: **YAMADA, Naoyoshi**
**29-4-204, Oyake Sakanotsujicho,**
**Yamashina-ku**
**Kyoto-shi, Kyoto 607(JP)**

(74) Representative: **Alber, Norbert (DE)**
**Albert-Rosshaupter-Strasse 65**
**W-8000 München 70 (DE)**

(54) **PIPERIDINE DERIVATIVE.**

(57) A 3,4,5-trihydroxypiperidine derivative of general formula (I), having a $\beta$-galactosidase inhibitory action and therefore usable as a carcinostatic agent, wherein R represents a $C_1$ to $C_{18}$ saturated or unsaturated hydrocarbon group which may be substituted with a linear, branched or cyclic group.

EP 0 536 402 A1

## TECHNICAL FIELD

The present invention relates to a piperidine derivative of the following general formula [I]

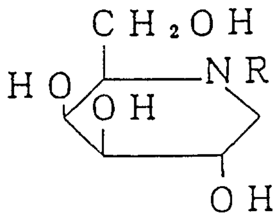

[I]

wherein R represents a straight-chain, branched or cyclic C1-18 saturated or unsaturated hydrocarbon group which may optionally be substituted.

## BACKGROUND ART

Bosmann et al. report that glucosidase activity and particularly β -galactosidase activity are elevated in the 3T3 fibroblast cells cancerized by RNA virus [H.B. Bosmann et al., Biochem. Biophys. Acta., 264, 339 (1972)]. Therefore, any β -galactosidase inhibitor has a potential of application as an anticancer agent or a cancer metastasis inhibitor and much research has been done on the subject as described in Japanese Patent Application No. 74090/1982, do. No. 31238/1978, do. No. 123135/1985, the Journal of Antibiotics, 28, 1006 (1975); do. 32 (3), 212 (1979), do. 32 (3), 217 (1979) and so on.

## DISCLOSURE OF INVENTION

It is not difficult to imagine that any β -galactosidase inhibitor exhibiting higher inhibitory activity is better than a less inhibitory one for use as an anticancer drug from the standpoint of dosage and adverse drug reaction, among other things.

Therefore, the inventors of the present invention did much research with emphasis on the discovery of compounds exhibiting more potent inhibitory activity than the known compounds. The intensive investigation by the inventors led to the finding that a 3,4,5-trihydroxypiperidine derivative of the above general formula [I], inclusive of a pharmacologically acceptable salt thereof, has high inhibitory activity and further to the development of the present invention. The compound of the present invention is a novel compound never described heretofore in the literature.

The straight-chain, branched or cyclic saturated or unsaturated hydrocarbon group mentioned above includes, among others, methyl, ethyl, n-propyl, isopropyl, butyl, isobutyl, sec-butyl, tert-butyl, n-heptyl, n-dodecyl, vinyl, allyl, isopropenyl, butenyl, heptenyl, decenyl, ethynyl, propynyl, cyclopropylmethyl, cyclobutylmethyl, cyclopentylmethyl, cyclohexylmethyl, cyclopropenylmethyl, cyclobutenylmethyl, cyclopentenylmethyl, cyclohexenylmethyl, benzyl and so on.

The substituent group includes not only such groups as methyl, ethyl, n-propyl, isopropyl, n-butyl, iso-butyl, sec-butyl, tert-butyl, heptyl, hydroxy, cyano, fluorine, chlorine, bromine, iodine, nitro, nitroso, formyl, acetyl, propionyl, hexanoyl, lauroyl, benzoyl, toluoyl, cynnamoyl, methoxy, ethoxy, propoxy, isopropoxy, butoxy, pentyloxy, phenoxy, benzyloxy, methylenedioxy, ethylenedioxy, carboxy, methoxycarbonyl, ethoxycarbonyl, propoxycarbonyl, formyloxy, acetoxy, benzoyloxy, amino, methylamino, dimethylamino, ethylamino, diethylamino, ethylmethylamino, acetylamino, benzoylamino, methoxycarbonylamino, ethoxycarbonylamino, propoxycarbonylamino, acetylmethylamino, sulfo, sulfamoyl, sulfoamino, carbamoyl, methylcarbamoyl, dimethylcarbamoyl, ethylcarbamoyl, diethylcarbamoyl, propylcarbamoyl, butylcarbamoyl, ureido, methylureido, dimethylureido, ethylureido, diethylureido, ethylmethylureido, phenylureido, thioureido, methylthioureido, dimethylthioureido, ethylthioureido, diethylthioureido, ethylmethylthioureido, phenylthioureido, guanidino, etc. but also such groups as phenyl, phenoxy, tosyl, cyclopropyl, cyclobutyl, cyclopentyl, cyclohexyl, cyclopropenyl, cyclobutenyl, cyclopentenyl, cyclohexenyl, pyrrolyl, thienyl, furyl, theonyl, pyridyl, piperidino, piperidyl, morpholyl, quinolyl, indolyl, phthalimido, anilino, etc. which may be respectively substituted by one or more of the above-mentioned substituent groups. Furthermore, O-β -D-glucopyranosyl, S-β -D-glucopyranosyl, etc. may also be mentioned as examples of the substituent.

The pharmacologically acceptable salt includes, among others, the corresponding metal salts such as lithium salt, sodium salt, potassium salt, calcium salt, etc., salts with organic bases, such as ethanolamine

salt, diethanolamine salt, etc., salts with inorganic acids, such as hydrochloride, sulfate, phosphate, etc., and salts with organic acids such as acetate, methanesulfonate, succinate, lactate, fumarate, maleate and so on.

The compound of the invention has a potential of application as an inhibitor of the enzymes involved in galactose processing in glycoprotein carbohydrate chains, and is expected to be of use as a research reagent for the study of glycoprotein carbohydrate chain processing or a drug having activities analogous to the antiviral activity [Fleet et al., FEBS Lett. 237, 128 (1988)] and cancer cell metastasis inhibitory activity [G. Pulverer et al., J. Cancer. Res. Clin. Oncol., 144, 217 1988)] or immunomodulating activity of castanospermine, 1-deoxynojirimycin and other α -glucosidase inhibitors associated with sugar chain processing.

Furthermore, the compound of the invention can be utilized for increasing the shelf-lives of food products, such as sugar, potato, fruit juice, beer, chocolate, jams and candies, by adding an effective amount of one or more species of the compound to such products.

The present invention is essentially directed to the very compound [I] described above.

The following is a partial listing of specific compounds of the present invention: N-methyl-1-deoxygalactostatin, N-ethyl-1-deoxygalactostatin, N-propyl-1-deoxygalactostatin, N-(2,3-dimethylbutyl)-1-deoxygalactostatin, N-(2,2,3-trimethylpentyl)-1-deoxygalactostatin, N-tert-butyl-1-deoxygalactostatin, N-n-pentyl-1-deoxygalactostatin, N-isopentyl-deoxygalactostatin, N-sec-pentyl-1-deoxygalactostatin, N-(3-ethyl-2-isopropylpentyl)-1-deoxygalactostain, N-hexyl-1-deoxygalactostatin, N-heptyl-1-deoxygalactostain, N-isohexyl-1-deoxygalactostatin, N-isoheptyl-1-deoxygalactostatin, N-octyl-1-deoxygalactostatin, N-isooctyl-1-deoxygalactostatin, N-decyl-1-deoxygalactostain, N-dodecyl-1-deoxygalactostatin, N-tetradecyl-1-deoxygalactostatin, N-hexadecyl-1-deoxygalactostatin, N-octadecyl-1-deoxygalactostatin, N-cyclopropylmethyl-1-deoxygalactostain, N-cyclopentylmethyl-1-deoxoygalactostatin, N-cyclohexylmethyl-1-deoxygalactostatin, N-(2-hydroxyethyl)-1-deoxygalactostatin, N-(3-hydroxypropyl)-1-deoxygalactostatin, N-(4-hydroxybutyl)-1-deoxygalactostatin, N-(5-hydroxypentyl)-1-deoxygalactostatin, N-(2-hydroxy-3-methylbtuyl)-1-deoxygalactostatin, N-(2,3-dihydroxypropyl)-1-deoxygalactostatin, N-(2-methoxyethyl)-1-deoxygalactostatin, N-(2-propoxyethyl)-1-deoxygalactostatin, N-(2-acetoxyethyl)-1-deoxygalactostatin, N-(4-benzoyloxybutyl)-1-deoxygalactostatin, N-(2-aminoethyl)-1-dexoygalactostatin, N-(2-dimethylaminoethyl)-1-deoxygalactostatin, N-(2-acetylaminoethyl)-1-deoxygalactostatin, N-(2-benzoylaminoethyl)-1-deoxygalactostatin, N-(2-propoxycarbonylaminoethyl)-1-deoxygalactostatin, N-(2-(N',N'-acetylmethyl)aminoethyl)-1-deoxygalactostatin, N-(2-(N'-methylureido)-ethyl)-1-deoxygalactostatin, N-(2-(N'-phenylureido)ethyl)-1-deoxygalactostatin, N-(2-(N'-methylthioureido)ethyl)-1-deoxygalactostatin, N-(2-(N'-phenylureido)ethyl)-1-deoxygalactostatin, N-(3-aminopropyl)-1-deoxygalactostatin, N-(3-acetylaminopropyl)-1-deoxygalactostatin, N-(3-benzoylaminopropyl)-1-deoxygalactostatin, N-(3-(N'-methylureido)propyl)-1-deoxygalactostatin, N-cynnamyl-1-deoxygalactostatin, 2-phenoxyethyl-1-deoxygalactostatin, N-(p-ethoxycarbonylphenoxy)ethyl)-1-deoxygalactostatin, N-(2-benzyloxyethyl)-1-deoxygalactostatin, N-(3-phenoxycarbonylpropyl)-1-deoxygalactostatin, N-(4-aminobutyl)-1-deoxygalactostatin, N-allyl-1-deoxygalactostatin, N-(2-butenyl)-1-deoxygalactostatin, N-(3-butenyl)-1-deoxygalactostatin, N-(5-hexenyl)-1-deoxygalactostatin, N-(9-decenyl)-1-deoxygalactostatin, N-carboxymethyl-1-deoxygalactostatin, N-(2-carboxyethyl)-1-deoxygalactostatin, N-ethoxycarbonylethyl-1-deoxygalactostatin, N-carbamoylmethyl-1-deoxygalactostatin, N-(N'-ethylcarbamoylmethyl)-1-deoxygalactostatin, N-(N'-butylcarbamoylmethyl)-1-deoxygalactostatin, N-(3-sulfopropyl)-1-deoxygalactostatin, N-(3-sulfamoylpropyl)-1-deoxygalactostatin, N-(o-carboxybenzyl)-1-deoxygalactostatin, N-(o-nitrobenzyl)-1-deoxygalactostatin, N-(5-bromo-2-hydroxybenzyl)-1-deoxygalactostatin, N-benzoylmethyl-1-deoxygalactostatin, N-(4-hydroxy-3-methoxybenzyl)-1-deoxygalactostatin, N-(2-propynyl)-1-deoxygalactostatin, N-(p-hydroxybenzyl)-1-deoxygalactostatin, N-(4-hydroxy-3-methoxy-5-nitrobenzyl)-1-deoxygalactostatin, N-(4-nitro-2-sulfobenzyl)-1-deoxygalactostatin, N-(2-hydroxy-4,6-dimethoxybenzyl)-1-deoxygalactostatin, N-(2-methylthiobenzyl)-1-deoxygalactostatin, disodium N-(2,4-disulfonatobenzyl)-1-deoxygalactostatin, N-(2-chloro-5-nitrobenzyl)-1-deoxygalactostatin, N-(2-chloro-6-nitrobenzyl)-1-deoxygalactostain, N-(4-chloro-3-nitrobenzyl)-1-deoxygalactostatin, N-(5-chloro-2-nitrobenzyl)-1-deoxygalactostatin, N-(o-bromobenzyl)-1-deoxygalactostatin, N-(p-bromobenzyl)-1-deoxygalactostatin, N-(o-chlorobenzyl)-1-deoxygalactostatin, N-(m-chlorobenzyl)-1-deoxygalactostatin, N-(p-chlorobenzyl)-1-deoxygalactostatin, N-(o-fluorobenzyl)-1-deoxygalactostatin, N-(m-fluorobenzyl)-1-deoxygalactostatin, N-(p-fluorobenzyl)-1-deoxygalactostatin, N-(o-nitrobenzyl)-1-deoxygalactostatin, N-(4-hydroxy-3-nitrobenzyl)-1-deoxygalactostatin, N-(5-hydroxy-2-nitrobenzyl)-1-deoxygalactostatin, N-(m-hydroxybenzyl)-1-deoxygalactostatin, N-(p-hydroxybenzyl)-1-deoxygalactostatin, N-(o-hydroxybenzyl)-1-deoxygalactostatin, N-(2,5-dihydroxybenzyl)-1-deoxygalactostatin, N-(3,4-dihydroxybenzyl)-1-deoxygalactostatin, N-(p-carboxybenzyl)-1-deoxygalactostatin, N-(3,4-methylenedioxybenzyl)-1-deoxy-galactostatin, N-(3-carboxy-4-hydroxybenzyl)-1-deoxy-galactostatin, N-(o-methylbenzyl)-1-deoxygalactostatin, N-(p-methylbenzyl)-1-deoxygalactostatin, N-(o-methoxy-benzyl)-1-deoxygalactostatin, N-(m-methoxybenzyl)-1-deoxygalactostatin, N-(4-hydroxy-3-methoxybenzyl)-1-deoxygalactostatin, N-(3-hydroxy-4-methoxybenzyl)-1-deoxygalactostatin, N-(3,4-dimethoxybenzyl)-1-deoxygalactostatin, N-(p-acetylaminobenzyl)-1-deoxygalactostatin, N-(2,5-dimethylbenzyl)-1-deoxygalactostatin, N-(o-ethoxybenzyl)-1-deoxygalactostatin, N-(2-methyl-4-methoxybenzyl)-1-deoxygalactostatin, N-(3,5-dimethoxybenzyl)-1-deoxygalactostatin, N-(p-dimethylaminobenzyl)-1-deoxygalactostatin, N-(3,4,5-trimethoxybenzyl)-1-deoxygalactostatin, N-(2,4,5-trimethoxybenzyl)-1-deoxygalactostatin, N-(2,3-epoxypropyl)-1-deoxygalactostatin, N-(3-phthalimidopropyl)-1-deoxygalactostatin, N-(2-phthalimidoethyl)-1-deoxygalactostatin, N-(2-pyridyl)methyl-1-deoxygalactostatin, N-(2-(S-$\beta$ -D-glucopyranosyl-2-mercapto)ethyl)-1-deoxygalactostatin, N-(2-(O-$\beta$ -D-glucopyronosyl)ethyl)-1-deoxygalactostatin, N-(2-furyl)methyl-1-deoxygalactostatin, N-(3-indolyl)methyl-1-deoxygalactostatin, N-(2-(5-bromothienyl))methyl-1-deoxygalactostatin, N-(2-pyrrolyl)methyl-1-deoxygalactostatin, N-(3-pyridyl)methyl-1-deoxygalactostatin, N-(4-pyridyl)methyl-1-deoxygalactostatin, N-benzyl-1-deoxygalactostatin and so on.

For administration of the compound of the present invention as a drug to man or other animals, it can be administered as it is or as formulated beforehand into a pharmaceutical composition containing 0.1 to 99.5%, preferably 0.5 to 90%, of the compound in a pharmaceutically acceptable, nontoxic and inert excipient.

The excipient mentioned above may be one or more solid, semisolid or liquid diluents, fillers and/or other formulation auxiliaries. Such a pharmaceutical composition is preferably administered in unit dosage forms. The pharmaceutical composition of the invention can be administered orally, into tissues, topically (transdermally etc.) or rectally. Of course, dosage forms suitable for the respective routes of administration should be employed. Particularly preferred is oral administration.

The dosage of the compound, as a $\beta$ -galactosidase inhibitor, should preferably be selected according to the patient's age, body weight and other characteristics, the route of administration and the nature and severity of the disease. The generally recommended dosage for an adult human is 0.1 mg to 3 g/body/day or preferably 1 mg to 100 mg/body/day. The required dosage may be somewhat less or more, depending on individual cases. The pharmaceutical composition is preferably administered in 1 to 3 divided doses.

For oral administration, either solid or liquid unit dosage forms, such as neat powders, powders, tablets, dragees, capsuls, granules, suspensions, solutions, syrups, drops, sublingual tablets, etc. can be provided.

Neat powders are manufactured by comminuting the active substance to size. Powders are manufactured by comminuting the active substance to size and admixing the resulting neat powder with a pharmaceutical excipient, such as an edible carbohydrate, e.g. starch, mannitol, etc., which has been similarly comminuted beforehand. If necessary, a corrigent, preservative, dispersant, colorant, perfume, etc. can also be incorporated.

Capsuls can be manufactured by preparing neat or formulated powders in the above manner or granules in the manner described hereinafter for tablets and, then, filling gelatin or other capsul shells with the powders or granules. Prior to the filling operation, a lubricant or fluidizing agent, such as colloidal silica, talc, magnesium stearate, calcium stearate, solid polyethylene glycol, etc., can be added, each in finely divided state, to said granules. An improvement in the efficacy of the encapsulated drug administered may be obtained by adding a disintegrator or solubilizer, such as carboxymethylcellulose, carboxymethylcellulose calcium, lowsubstituted hydroxypropylcellulose, crosscarmellose sodium, carboxystarch sodium, calcium carbonate, sodium carbonate and so on.

Soft capsules can be obtained by suspending a finely divided powder of the drug in a mixture of vegetable oil, polyethylene glycol, glycerin and surfactant and sealing the suspension in flexible gelatin sheets. Tablets can be manufactured by preparing a powdery composition, processing it into granules or slags, and after addition of a disintegrator or lubricant, compression-molding the mixture. A powdery composition can be prepared by mixing the pulverized drug with said diluent and base, with or without addition of a binder (e.g. carboxymethylcellulose sodium, hydroxypropylcellulose, methylcellulose, hydroxypropylmethylcellulose, gelatin, polyvinylpyrrolidone, polyvinyl alcohol, etc.), a dissolution retardant (e.g. paraffin, wax, hydrogenated castor oil, etc.), a reabsorption agent (e.g. a quaternary salt) and/or an adsorbent (e.g. bentonite, kaolin, dicalcium phosphate, etc.). The powdery composition can be granulated by wetting it with a binder, such as a syrup, starch paste, gum arabic, a cellulose or polymer solution or the like and, then, passing it through a sieve by force. Instead of such a granulation process, the composition may first be tableted and the resulting slags of crude form are comminuted into granules.

To the granules which are manufactured in the above manner, an appropriate lubricant, such as stearic acid, stearates, talc, mineral oil, etc., can be added for preventing the interadhesion of individual granules. The thus-lubricated composition is then compression-molded.

Instead of being processed through said granulation and slagging steps, the drug may be admixed with a free-flowing inert carrier and directly compression-molded. It is also possible to utilize a transparent or translucent protective coating consisting in a hermetically sealing shellac film, a sugar or polymeric coating or a polished wax coating.

Other oral dosage forms such as solutions, syrups and elixirs can also be provided in unit dosage forms so that the drug may be delivered in constant quantities. Syrups can be manufactured by dissolving the compound in an appropriate flavored aqueous medium, while elixirs can be manufactured using a nontoxic alcoholic vehicle. Suspensions can be formulated by dispersing the compound in a nontoxic vehicle. Solubilizers and emulsifiers (e.g. ethoxylated isostearyl alcohol, polyoxyethylene sorbitol ester, etc.), preservatives, flavorants (e.g. peppermint oil, saccharin, etc.) and other agents can also be added, where necessary.

If necessary, such a unit dosage form for oral administration can be microencapsulated. The dosage unit may also be coated or embedded in a polymer, wax or the like for prolonged action or sustained release.

For administration into tissues, liquid unit dosage forms for subcutaneous, intramuscular or intravenous administration, such as solutions and suspensions, can be utilized. Thus, these preparations can be manufactured by suspending or dissolving a predetermined amount of the active compound in an injectable nontoxic liquid vehicle, such as an aqueous or oily medium, and sterilizing the resulting suspension or solution. An alternative procedure comprises filling vials with a predetermined amount of the active compound, sterilizing the filled vials and sealing them. For extemporaneous reconstitution or blending, there may be provided a spare vial and vehicle accompanying a powdered or lyophilized preparation of the active ingredient. For isotonization, a nontoxic salt or a solution of the salt may be added to an injectable composition. Moreover, stabilizers, preservatives, emulsifiers and the like may also be employed.

For rectal administration, suppositories can be provided by formulating the active compound with a water-soluble or -insoluble solid, such as polyethylene glycol, cacao butter, semisynthetic oily base (e.g. Witepsol(trademark)), a higher ester (e.g. myristyl palmitate), or a mixture thereof.

(Example of Synthesis)

The compound of the present invention can be synthesized by the method for reductive substitution of the hydrogen attached to the nitrogen of 1-deoxygalactostatin [II] in the per se known manner , for example by means of a carbonyl compound and a hydrogen donating reducing agent, the method for direct substitution using a reagent having any of said various substituents, or the method comprising acylating the nitrogen of [II] and reducing the acylated compound in the per se known manner. To be specific, the following processes may be mentioned.

Reductive substitution

The compound of the present invention can be obtained in accordance with the following reaction schema, for example by reacting compound [II] with a ketone or an aldehyde and a suitable reducing agent, such as an alkali metal cyanoborohydride, a dialkylaminoborane or an alkali metal borohydride, specifically sodium cyanoborohydride ($NaBH_3CN$), sodium borohydride/trifluoroacetic acid, or Raney nickel/hydrogen in an appropriate solvent, e.g. water-alcohol, at 0 to 100 ° C .

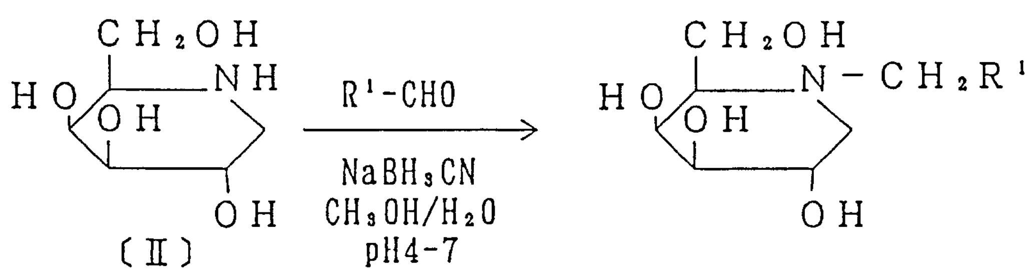


[II]

(wherein R1 represents hydrogen or hydroxyl or has the same meaning as R defined hereinabove)

The Leuekart-Wallach reaction can also be employed.

Direct substitution

The synthesis of the compound of the present invention by direct substitution of the hydrogen attached to the nitrogen with a substituent group can be carried out in accordance with the following reaction schema, for example reacting compound [II] with an alkylating agent (Z-R) and an appropriate base, such

as potassium carbonate, in an appropriate solvent, such as N,N-dimethylformamide (hereinafter referred to as DMF) at 0 to 100 ° C .

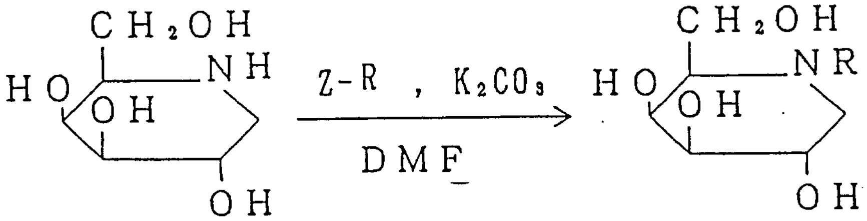


(II)

(wherein R is as defined hereinbefore; Z represents a group which is ready to leave and conventional for an alkylating agent, for example a halogen atom such as chlorine, bromine or iodine).

Reduction of the acyl compound

The synthesis of the compound of the present invention by reduction of the acyl compound is carried out in accordance with the following reaction schema, for example by arylating compound [II] with an acyl halide ($R^2$-CO-X) or the corresponding anhydride ($R^2$-CO-O-CO-$R^2$) in an appropriate solvent, such as water, water-alcohol or DMF, and then reducing it with an appropriate reducing agent such as lithium aluminum hydride (LiAlH4).

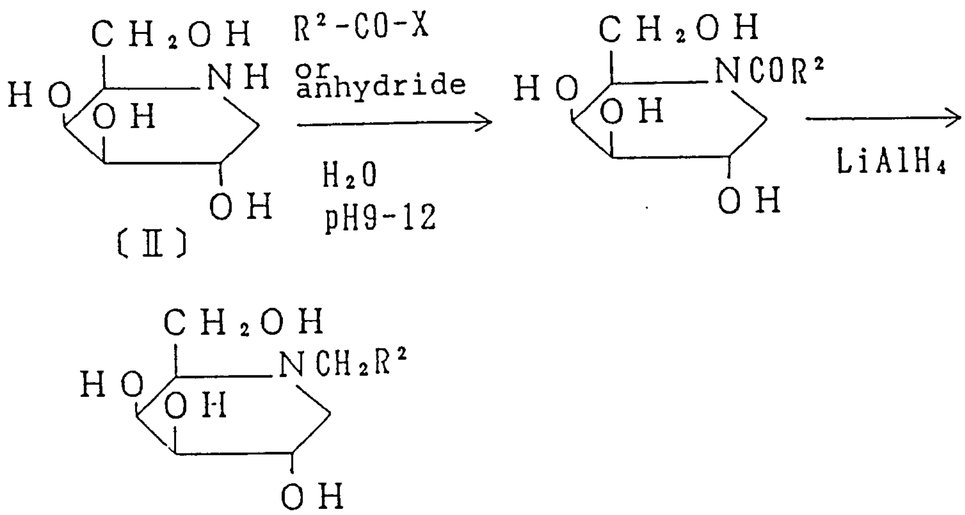


(II)

(wherein R2 has the same meaning as R defined hereinbefore; X represents halogen, such as chlorine, bromine or iodine)

BEST MODE FOR CARRYING OUT THE INVENTION

The following examples, reference examples and experimental example are intended to describe the invention in further detail and should by no means be construed as defining the metes and bounds of the invention.

Reference Example 1 Synthesis of N-benzyloxycarbonylmoranoline

In a mixture of 160 ml of water and 160 ml of chloroform were dissolved 16.3 g (0.1 mol) of moranoline and 8.4 g (0.1 mol) of sodium hydrogen carbonate followed by addition of 20.47 g (0.12 mol) of benzyloxycarbonyl chloride with ice-cooling and the mixture was vigorously stirred. After 8 hours, 0.84 g (0.01 mol) of sodium hydrogen carbonate and 5.12 g (0.03 mol) of benzyloxycarbonyl chloride were further added with ice-cooling and reacted for 6 hours. The aqueous layer was adjusted to pH 5-6 and extracted 3

times with one volume each of chloroform. The aqueous layer was then dried. To the resulting solid was added ethyl acetate-ethanol (1:1) and the insoluble matter was filtered off. The filtrate was dried to give 30 g (quantitative) of the title compound as oil.

Reference Example 2 Synthesis of N-benzyloxycarbonyl-4,6-O-benzylidenemoranoline

To 290 ml of DMF were added 29 g (97 mmols) of the compound of Reference Example 1, 3.34 g (19 mmols) of anhydrous toluenesulfonic acid, 29.4 g (194 mmols) of benzaldehyde dimethyl acetal and 29 g of activated calcium sulfate and the mixture was stirred at 30 °C for 24 hours. The reaction mixture was neutralized with a strongly basic ion exchange resin (Diaion SA-11A, $OH^-$-form) and the insoluble matter was filtered off. The filtrate was dried and crystallized from ethyl acetate - hexane to give 29 g (yield 72%) of the title compound as an amorphous substance.
Melting point: 134-138 °C .

Reference Example 3 Synthesis of N-benzyloxycarbonyl-2,3-di-O-benzyl-4,6-O-benzylidenemoranoline

To 1,000 ml of DMF were added 27 g (70 mmols) of the compound of Reference Example 2 and 21g (875 mmols) of 60% sodium hydride and the mixture was stirred for 30 minutes. Then, 170 g (995 mmols) of benzyl bromide was added dropwise with ice-cooling and the mixture was allowed to stand for 2 days. Thereafter, methanol was added and the mixture was dried. The resulting oil was dissolved in chloroform and extracted with water a few times and the chloroform layer was dried. The oily substance thus obtained was subjected to silica-gel column chromatography (Wakogel C-200) and elution was carried out with hexane-ethyl acetate (9:1) to give 37.5 g (yield 94%) of the title compound as oil.

Reference Example 4 Synthesis of N-benzyloxycarbonyl-2,3-di-O-benzylmoranoline

In a mixture of 320 ml of acetic acid and 80 ml of water was dissolved 36.5 g (64 mmols) of the compound of Reference Example 3 and the mixture was stirred at 60 °C for 6 hours. Thereafter, the reaction mixture was dried to give 36.3 g of an oil containing the title compound.

Reference Example 5 Synthesis of N,6-O-carbamoyl-2,3-di-O-benzylmoranoline

In a mixture of 360 ml of methanol and 40 ml of water were dissolv-ed 36.3 g of the above oil containing the compound of Reference Example 4 and 40 g of potassium carbonate and the mixture was stirred at 60 °C for 4 hours. After completion of stirring, the reaction mixture was dried and the resulting solid was dissolved in chloroform and extracted with a few portions of water. The chloroform layer was dried and the residue was crystallized from ethyl acetate - hexane to give 19 g of an amorphous substance.
Melting point 110-110 °C .

Reference Example 6 Synthesis of N,6-O-carbamoyl-2,3-di-O-benzyl-4-O-mesylmoranoline

In 200 ml of acetone were dissolved 19 g (51 mmols) of the compound of Reference Example 5 and 15.6 g (154 mmols) of triethylamine followed by dropwise addition of 9.8 g (85 mmol) of mesyl chloride with ice-cooling, and the mixture was stirred for 30 minutes. After completion of stirring, the reaction mixture was dried and distributed between 0.1N hydrochloric acid and ethyl acetate. The ethyl acetate layer was dried. This ethyl acetate solution was distributed between 0.1 N sodium hydrogen carbonate and chloroform and the chloroform layer was dried. The residue was crystallized from ethyl acetate, chloroform and hexane to give 22.6 g (yield 98%) of the title compound.
Melting point 197-199 °C .

Reference Example 7 Synthesis of N,6-O-carbamoyl-2,3-di-O-benzyl-4-O-benzoyl-1-deoxygalactostatin

In 30 ml of DMF were dissolved 22.1 g (49 mmols) of the compound of Reference Example 6 and 7.54 g (58 mmols) of lithium benzoate and the mixture was stirred at 100 °C for 2 days. After completion of stirring, the reaction mixture was dried and distributed between 0.2N sodium hydrogen carbonate and ethyl acetate. The ethyl acetate layer was dried to give 24.5 g (quantitative) of the title compound as oil. This oil was treated with diethyl ether to give an amorphous substance. Melting point 135-137 °C .

Reference Example 8 Synthesis of N,6-O-carbamoyl-2,3-di-O-benzyl-1-deoxygalactostatin

In a mixture of 450 ml of methylene chloride and 100 ml of methanol was dissolved 24 g (50 mmols) of the compound of Reference Example 7 followed by addition of 5 ml of 10N sodium hydroxide, and the mixture was stirred at 50°C for 3 hours. After completion of stirring, the reaction mixture was neutralized with concentrated hydrochloric acid, dried and distributed between chloroform and water. The chloroform layer was dried to give 20.9 g of an oil containing the title compound.

Reference Example 9 Synthesis of 2,3-di-O-benzyl-1-deoxygalactostatin

In a mixture of 320 ml of methanol and 80 ml of water were dissolved 20 g of the above oil containing the compound of Reference Example 8 and barium hydroxide octahydrate and the mixture was refluxed with stirring for 4 hours. After completion of stirring, dry ice was added to the reaction mixture and the mixture was centrifuged at 8,500 rpm. The resulting precipitate was washed with methanol and recentrifuged. The supernatants were combined, dried and distributed between 0.1N hydrochloric acid and chloroform. The hydrochloric acid layer was made weakly basic with sodium carbonate and the title compound was extracted with ethyl acetate. The ethyl acetate layer was dried and crystallized from ethyl acetate - hexane to give 11.82 g of plates.
Melting point 126-128°C

Reference Example 10 Synthesis of 1-deoxygalactostatin hydrochloride

In liquid ammonia was dissolved 10 g of the compound obtained in Reference Example 9 followed by addition of 2.8 g of sodium metal, and the mixture was stirred in acetone-dry ice for 30 minutes. Then, ammonium chloride was added until the blue color of the reaction mixture had disappeared. The ammonia was evaporated off and the solid residue was applied to a column of strongly acidic ion exchange resin (Dowex 50WX-2, $H^+$-form), and after the column was washed with water, elution was carried out with 1N aqueous ammonia. The eluate was dried and applied to a column of strongly basic ion exchange resin (Diaion SA-11A, $OH^-$-form) and the effluent obtained with water was dried. The residual oily substance was dissolved in ethanol and the solution was made weakly acidic to provide 5.26 g (yield 90%) of the title compound as crystals. Melting point 237-239°C .
$[\alpha]_D$ 54.96 (20°C , C=0.997, $H_2O$)

| Elemental Analysis for $C_6H_{14}ClNO_4$ | | | |
|---|---|---|---|
| Calcd. (%): | C:36.10 | H:7.07 | N:7.02 |
| Found (%): | C:35.97 | H:7.09 | N:7.04 |

Example 1 Synthesis of N-methyl-1-deoxygalactostatin

In a mixture of 12.5 ml of methanol and 2.5 ml of water were dissolved 0.5 g (2.5 mmols) of 1-deoxygalactostatin hydrochloride, 0.64 g (7.5 mmols) of 35% formalin and 0.16 g (2.5 mmols) of sodium cyanoborohydride and the mixture was adjusted to pH4- 5 with glacial acetic acid and stirred at room temperature for 2 hours. Thereafter, the mixture was applied to a column of strongly acidic ion exchange resin (Dowex 50WX-2, $H^+$-form), and after the ion exchange resin was washed with methanol, elution was carried out with methanol-concentrated aqueous ammonia=10:1. The eluate was concentrated to dryness in a rotary evaporator and the residue was applied to a strongly basic ion exchange resin (Diaion SA-11A, $OH^-$- form) and the aqueous effluent was concentrated to dryness. The residue was recrystallized from ethanol to give 0.34 g of the compound of the present invention. Melting point 164-166°C .
$[\alpha]_D$ -3.27 (20°C , C=1.037, $H_2O$)

| Elemental analysis for $C_7H_{15}NO_4$ | | | |
|---|---|---|---|
| Calcd. (%): | C:47.45 | H:8.53 | N:7.90 |
| Found (%): | C:47.44 | H:8.51 | N:7.94 |

Example 2 Synthesis of N-ethyl-1-deoxygalactostatin

Using acetaldehyde in lieu of formalin, the synthetic procedure of Example 1 was otherwise repeated. Melting point 159-161 °C.
$[\alpha]_D$ -21.31 (20 °C, C=1.032, $H_2O$)

| Elemental analysis for $C_8H_{17}NO_4$ | | | |
|---|---|---|---|
| Calcd. (%): | C:50.25 | H:8.96 | N:7.32 |
| Found (%): | C:49.96 | H:8.85 | N:7.31 |

Example 3 Synthesis of N-n-propyl-1-deoxygalactostatin

Using propionaldehyde in lieu of formalin, the synthetic procedure of Example 1 was otherwise repeated. Melting point 120-122 °C.
$[\alpha]_D$ -27.00 (20 °C, C=0.985, $H_2O$)

| Elemental analysis for $C_9H_{19}NO_4$ | | | |
|---|---|---|---|
| Calcd. (%): | C:52.67 | H:9.33 | N:6.82 |
| Found (%): | C:52.52 | H:9.21 | N:6.87 |

Example 4 Synthesis of N-isobutyl-1-deoxygalactostatin

Using isobutanal in lieu of formalin, the synthetic procedure of Example 1 was otherwise repeated. The product compound was crystallized as the hydrochloride.
Melting point 142-145 °C.
$[\alpha]_D$ 1.93 (20 °C, C=0.516, $H_2O$)

| Elemental analysis for $C_{10}H_{21}NO_4 \cdot HCl$ | | | |
|---|---|---|---|
| Calcd. (%): | C:46.97 | H:8.67 | N:5.48 |
| Found (%): | C:46.81 | H:8.67 | N:6.46 |

Example 5 Synthesis of N-n-heptyl-1-deoxygalacto-statin

Using heptanal in lieu of formalin, the synthetic procedure of Example 1 was otherwise repeated. Melting point 125-127 °C.
$[\alpha]_D$ -25.92 (20 °C, C=1.003, MeOH)

| Elemental analysis for $C_{13}H_{27}NO_4$ | | | |
|---|---|---|---|
| Calcd. (%): | C:59.74 | H:10.41 | N:5.36 |
| Found (%): | C:59.25 | H:10.41 | N:5.37 |

Example 6 Synthesis of N-n-dodecyl-1-deoxygalactostatin

Using dodecanal in lieu of formalin, the synthetic procedure of Example 1 was otherwise repeated. Melting point 124-128 °C.
$[\alpha]_D$ -14.41 (20 °C, C=0.999, DMSO)

| Elemental analysis for $C_{18}H_{37}NO_4$ | | | |
|---|---|---|---|
| Calcd. (%): | C:65.22 | H:11.25 | N:4.23 |
| Found (%): | C:64.79 | H:10.91 | N:4.22 |

Example 7 Synthesis of N-(3-phenylpropyl)-1-deoxygalactostatin

Using 3-phenylpropanal in lieu of formalin, the synthetic procedure of Example 1 was otherwise repeated. Melting point 100-104 °C .
$[\alpha]_D$ -25.94 (20 °C , C = 0.501, MeOH)

| Elemental analysis for $C_{15}H_{23}NO_4$ | | | |
|---|---|---|---|
| Calcd. (%): | C:64.04 | H:8.24 | N:4.98 |
| Found (%): | C:64.01 | H:8.35 | N:5.02 |

Example 8 Synthesis of N-p-chlorobenzyl-1-deoxygalactostatin

Using p-chlorobenzaldehyde in lieu of formalin, the synthetic procedure of Example 1 was otherwise repeated and the product was crystallized as the hydrochloride. Melting point 101-104 °C .
$[\alpha]_D$ 12.59 (20 °C , C = 1.000, $H_2O$)

| Elemental analysis for $C_{15}H_{23}NO_4 \cdot HCl \cdot 1/2H_2O$ | | | |
|---|---|---|---|
| Calcd. (%): | C:46.86 | H:6.05 | N:4.20 |
| Found (%): | C:46.75 | H:6.22 | N:4.13 |

Example 9 Synthesis of N-allyl-1-deoxygalactostatin

In 5 ml of DMF were suspended 0.3 g (1.5 mmols) of 1-deoxygalactostatin hydrochloride and 0.21 g (1.7 mmols) of anhydrous potassium carbonate followed by addition of 0.2 g(1.65 mmols) of allyl bromide with ice-cooling and the mixture was stirred at room temperature for 12 hours. After completion of stirring, the salt was filtered off and the filtrate was subjected to resin treatment as in Example 1 to give 0.19 g of the title compound of the invention. Melting point 144-146 °C .
$[\alpha]_D$ -14.69 (20 °C , C = 1.007, $H_2O$)

| Elemental analysis for $C_9H_{17}NO_4$ | | | |
|---|---|---|---|
| Calcd. (%): | C:53.19 | H:8.43 | N:6.89 |
| Found (%): | C:53.00 | H:8.49 | N:6.79 |

Example 10 Synthesis of N-cinnamyl-1-deoxygalactostatin

Using cinnamyl bromide in lieu of allyl bromide, the synthetic procedure of Example 9 was otherwise repeated. Melting point 66-69 °C .
$[\alpha]_D$ -40.63 (20 °C , C = 0.507, MeOH)

| Elemental analysis for $C_{15}H_{21}NO_4 \cdot 1/2H_2O$ | | | |
|---|---|---|---|
| Calcd. (%): | C:62.48 | H:7.69 | N:4.86 |
| Found (%): | C:62.56 | H:7.68 | N:4.89 |

Example 11 Synthesis of N-methoxyethyl-1-deoxygalactostatin

Using acetaldehyde, the synthetic procedure of Example 1 was otherwise repeated. Melting point 110-111°C.
$[\alpha]_D$ -14.42 (20°C, C=1.040, $H_2O$)
FAB-MAS m/z 222 ($M^+$ +1)

| Elemental analysis for $C_9H_{19}NO_5$ | | | |
|---|---|---|---|
| Calcd. (%) : | C:48.86 | H:8.66 | N:6.33 |
| Found (%): | C:48.43 | H:8.83 | N:6.37 |

Example 12 Synthesis of N-(p-phenylbenzyl)-1-deoxygalactostatin

Using phenylbenzaldehyde, the synthetic procedure of Example 1 was otherwise repeated. Melting point 177-178°C.
$[\alpha]_D$ -24.03 (20°C, C=0.491, DMSO)
FAB-MAS m/z 330 ($M^+$ +1)

| Elemental analysis for $C_{19}H_{23}NO_4 \cdot 1/4H_2O$ | | | |
|---|---|---|---|
| Calcd. (%): | C:68.35 | H:7.09 | N:4.19 |
| Found (%): | C:68.71 | H:7.35 | N:4.20 |

Example 13 Synthesis of N-n-pentylnyl-1-deoxygalactostatin

Using n-pentanal in lieu of formalin, the synthetic procedure of Example 1 was otherwise repeated. Melting point 115-116°C.
$[\alpha]_D$ -26.30 (20°C, C=0.517, $H_2O$)
FAB-MAS m/z 234 ($M^+$ +1)

| Elemental analysis for $C_{11}H_{23}NO_4$ | | | |
|---|---|---|---|
| Calcd. (%): | C:56.63 | H:9.94 | N:6.00 |
| Found (%): | C:56.35 | H:9.63 | N:6.06 |

Example 14 Synthesis of N-p-methoxybenzyl-1-deoxygalactostatin

Using p-anisaldehyde in lieu of formalin, the synthetic procedure of Example 1 was otherwise repeated. Melting point 122-124°C.
$[\alpha]_D$ -26.62 (20°C, C=0.586, $H_2O$)
FAB-MAS m/z 284 ($M^+$ +1)

| Elemental analysis for $C_{14}H_{21}NO_4$ | | | |
|---|---|---|---|
| Calcd. (%): | C:59.35 | H:7.47 | N:4.94 |
| Found (%): | C:59.05 | H:7.43 | N:4.91 |

Example 15 Synthesis of N-p-methylthiobenzyl-1-deoxygalactostatin

Using p-methylthiobenzaldehyde in lieu of formalin, the synthetic procedure of Example 1 was otherwise repeated. Melting point 120-123°C.

FAB-MAS m/z 300 ($M^+$ +1)

| Elemental analysis for $C_{14}H_{21}NO_4S \cdot 1/2H_2O$ | | | |
|---|---|---|---|
| Calcd. (%):<br>Found (%): | C:54.52<br>C:54.38 | H:7.20<br>H:6.91 | N:4.54<br>N:4.72 |

Example 16 Synthesis of N-phenylethyl-1-deoxygalactostatin

Using phenylacetaldehyde in lieu of formalin, the synthetic procedure of Example 1 was otherwise repeated. Melting point 188-190 °C.
FAB-MAS m/z 268 ($M^+$ +1)

| Elemental analysis for $C_{14}H_{21}NO_4$ | | | |
|---|---|---|---|
| Calcd. (%):<br>Found (%): | C:62.90<br>C:62.57 | H:7.93<br>H:8.01 | N:5.24<br>N:5.32 |

Example 17 Synthesis of N-(1'-deoxygalactitoyl)-1-deoxygalactostatin

Using D-galactose in lieu of formalin, the synthetic procedure of Example 1 was otherwise repeated. Melting point 79-82 °C.
$[\alpha]_D$ -17.60 (20 °C, C=0.284, $H_2O$)
FAB-MAS m/z 328 ($M^+$ +1)

| Elemental analysis for $C_{12}H_{25}NO_9 \cdot 2H_2O$ | | | |
|---|---|---|---|
| Calcd. (%):<br>Found (%): | C:39.67<br>C:39.70 | H:8.04<br>H:8.32 | N:3.85<br>N:4.15 |

Example 18 Synthesis of N-(p-acetamidobenzyl)-1-deoxygalactostatin

Using p-acetaminobenzaldehide in lieu of formalin, the synthetic procedure of Example 1 was otherwise repeated. Melting point 95-96 °C.
$[\alpha]_D$ -20.73 (20 °C, C=0.492, $H_2O$)
FAB-MAS m/z 311 ($M^+$ +1)

| Elemental analysis for $C_{15}H_{22}N_2O_5 \cdot 3/4H_2O$ | | | |
|---|---|---|---|
| Calcd. (%):<br>Found (%): | C:55.63<br>C:55.89 | H:7.31<br>H:7.55 | N:8.65<br>N:8.64 |

Example 19 Synthesis of N-phenylpropyl-1-deoxygalactostatin

Using phenylpropanal in lieu of formalin, the synthetic procedure of Example 1 was otherwise repeated. Melting point 100-104 °C.
$[\alpha]_D$ -25.94 (20 °C, C=0.501, MeOH)
FAB-MAS m/z 282 ($M^+$ +1)

| Elemental analysis for $C_{15}H_{23}NO_4$ | | | |
|---|---|---|---|
| Calcd. (%):<br>Found (%): | C:64.04<br>C:64.01 | H:8.24<br>H:8.35 | N:4.98<br>N:5.02 |

Example 20 Synthesis of N-cyclohexylmethyl-1-deoxygalactostatin

Using cyclohexanecarboxaldehyde in lieu of formalin, the synthetic procedure of Example 1 was otherwise repeated. Melting point 72-73 °C .
$[\alpha]_D$ -39.72 (20 °C , C = 0.715, $H_2O$)
FAB-MAS m/z 260 ($M^+$ + 1)

| Elemental analysis for $C_{13}H_{25}NO_4 \cdot 1/4H_2O$ | | | |
|---|---|---|---|
| Calcd. (%):<br>Found (%): | C:59.18<br>C:58.89 | H:9.74<br>H:9.77 | N:5.31<br>N:5.29 |

Example 21 Synthesis of N-(3'-methylthiopropyl)-1-deoxygalactostatin

Using methylthiopropionaldehyde in lieu of formalin, the synthetic procedure of Example 1 was otherwise repeated. Melting point 121-124 °C .
$[\alpha]_D$ -16.00 (20 °C , C = 0.550, $H_2O$)
FAB-MAS m/z 252 ($M^+$ + 1)

| Elemental analysis for $C_{10}H_{21}NO_4S \cdot 1/2H_2O$ | | | |
|---|---|---|---|
| Calcd. (%):<br>Found (%): | C:46.13<br>C:46.11 | H:8.51<br>H:8.21 | N:5.38<br>N:5.30 |

Example 22 Synthesis of N-(3'-methyl-4'-methoxybenzyl)-1-deoxygalactostatin

Using 3-methyl-4-methoxybenzaldehyde in lieu of formalin, the synthetic procedure of Example 1 was otherwise repeated. Melting point 185-187 °C .
FAB-MAS m/z 298 ($M^+$ + 1)

| Elemental analysis for $C_{15}H_{23}NO_5$ | | | |
|---|---|---|---|
| Calcd. (%):<br>Found (%): | C:60.59<br>C:60.48 | H:7.80<br>H:7.82 | N:4.71<br>N:4.66 |

Example 23 Synthesis of N-n-butyl-1-deoxygalactostatin

Using n-butyl bromide in lieu of allyl bromide, the synthetic procedure of Example 9 was otherwise repeated. Melting point 121-123 °C .
$[\alpha]_D$ -23.90 (20 °C , C = 0.502, $H_2O$)
FAB-MAS m/z 220 ($M^+$ + 1)

| Elemental analysis for $C_{10}H_{21}NO_4$ | | | |
|---|---|---|---|
| Calcd. (%):<br>Found (%): | C:54.77<br>C:54.33 | H:9.65<br>H:9.56 | N:6.39<br>N:6.36 |

Example 24 Synthesis of N-(3-carboxypropyl)-1-deoxygalactostatin

Using ethyl 3-bromopropionate in lieu of allyl bromide, the synthetic procedure of Example 9 was otherwise repeated. Melting point 105-107 °C .
$[\alpha]_D$ 8.10 (20 °C , C = 0.148, $H_2O$)
FAB-MAS m/z 250 ($M^+$ + 1)

| Elemental analysis for $C_{10}H_{19}NO_6 \cdot H_2O$ | | | |
|---|---|---|---|
| Calcd. (%) : | C:44.94 | H:7.92 | N:5.24 |
| Found (%): | C:44.58 | H:7.97 | N:5.24 |

Experimental Example

β -galactosidase inhibitory activity

The assay of this activity was carried out by permitting β -galactosidase to act on the substrate O-nitrophenyl-β -D-galactopyranose and quantitating O-nitrophenol, which was released on hydrolysis, colorimetrically. Thus, a mixture of 0.9 ml of 100 mM acetate buffer (pH 5.0), 0.1 ml of a solution of the test substance (dissolved in 100 mM acetate buffer at pH 5.0) and 0.5 ml of a 20 mM substrate solution was previously warmed at 37 °C for 5 hours and, then, dissolved in 10 mM acetate buffer at pH 5.0. Then, 0.5 ml of a solution of β -galactosidase was added and the reaction was conducted at 37 °C for 15 minutes. The absorbance (A) at 420 nm was then measured. The absorbance (B) of a reaction mixture not containing the test substance was also measured and using the equation of % inhibition = (B - A)/B x 100, the concentration of the test substance causing 50% inhibition of β -galactosidase activity ($IC_{50}$) was determined. The results of two experiments (Test Example 1 and Test Example 2) are shown in Table 1. The β -galactosidase derived from Aspergillus sp. was used. It is clear that the compound of the present invention has high β -galactosidase inhibitory activity.

Table 1

| β -Galactosidase inhibitory activity | | | |
|---|---|---|---|
| Test Example 1 | | Test Example 2 | |
| Example No. | $IC_{50}$ (ng/ml) | Example No. | $IC_{50}$ (ng/ml) |
| 1 | 15 | 11 | 59 |
| 2 | 82 | 12 | 166 |
| 3 | 44 | 13 | 113 |
| 4 | 40 | 14 | 391 |
| 5 | 32 | 15 | 393 |
| 6 | 23 | 16 | 21 |
| 7 | 88 | 17 | 49 |
| 8 | 180 | 18 | 340 |
| 9 | 187 | 20 | 37 |
| 10 | 175 | 21 | 49 |
| Control (1-deoxygalactostatin hydrochloride) | 451 | 22 | 359 |
| | | 23 | 154 |
| | | 24 | 351 |
| | | Control (1-deoxygalactostatin hydrochloride) | 440 |

## Claims

**1.** A 3,4,5-trihydroxypiperidine derivative of the following general formula [I] or a pharmaceutically acceptable salt thereof.

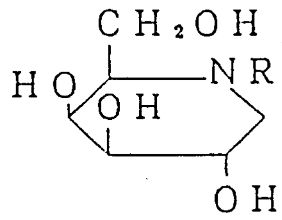

[I]

wherein R represents a straight-chain, branched or cyclic C1-18 saturated or unsaturated hydrocarbon group which may optionally be substituted.

# INTERNATIONAL SEARCH REPORT

International Application No PCT/JP91/00866

**I. CLASSIFICATION OF SUBJECT MATTER** (if several classification symbols apply, indicate all) [6]

According to International Patent Classification (IPC) or to both National Classification and IPC

Int. $Cl^5$ C07D211/46, A61K31/445

**II. FIELDS SEARCHED**

Minimum Documentation Searched [7]

| Classification System | Classification Symbols |
|---|---|
| IPC | C07D211/46, A61K31/445 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 62-242663 (Bayer AG), October 23, 1987 (23. 10. 87) & EP, A2, 240,868 | 1 |
| A | JP, A, 61-200967 (Bayer AG), September 5, 1986 (05. 09. 86) & EP, A2, 193,770 | 1 |
| A | JP, A, 57-134465 (Bayer AG), August 19, 1982 (19. 08. 82) & EP, A1, 55431 | 1 |
| A | JP, A, 64-31764 (Bayer AG), February 2, 1989 (02. 02. 89) & EP, A2, 298,350 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 30, 1991 (30. 08. 91) | September 17, 1991 (17. 09. 91) |
| **International Searching Authority** | **Signature of Authorized Officer** |
| Japanese Patent Office | |